# EUROPEAN PATENT APPLICATION

(11) **EP 4 595 952 A1**
(43) Date of publication of application: **06.08.2025**
(21) Application number: 23873093.1
(22) Date of filing: 26.09.2023
(51) Int. Cl.: A61K 9/08, A61K 47/36, A61K 47/40, A61K 9/00, A61K 31/498, A61P 27/02, A61P 27/06

(54) **OPHTHALMIC COMPOSITION COMPRISING BRIMONIDINE**

(30) Priority: 29.09.2022 KR 20220124725
(71) Applicant: Taejoon Pharmaceutical Co., Ltd., Seoul 04401 (KR)
(72) Inventor: LEE, Joon Youb, Seoul 04401 (KR); RYU, Sang Rok, Suwon-si Gyeonggi-do 16222 (KR); HAN, Byung Hyun, Yongin-si Gyeonggi-do 16840 (KR); GO, Hee Kyoung, Suwon-si Gyeonggi-do 16483 (KR)
(74) Representative: HGF
(86) International application number: PCT/KR2023/014800
(87) International publication number: WO 2024/071979

(57) **Abstract**

The present invention relates to an ophthalmic composition including brimonidine or salts thereof, SBE-β-CD or salts thereof, and xanthan gum, thereby showing excellent safety, stability and therapeutic effects.

## Description

### Technical Field

The present invention relates to an ophthalmic composition including brimonidine or salts thereof.

### Background

Brimonidine is an alpha-2-adrenergic agonist which is a drug for lowering an intraocular pressure of patients with glaucoma and/or ocular hypertension.

As a representative ophthalmic preparation containing brimonidine, there is Alphagan^{®}-P (Allergan, Inc.) containing brimonidine tartrate in an amount of 0.15%, which is prescribed to be administered three times a day.

Due to the properties of the ophthalmic preparation, it is preferable to have a pH in a biocompatible range capable of minimizing irritation to eyeballs and needs to have storage stability such that no discoloration or precipitation occurs. However, brimonidine has a property in which solubility thereof rapidly decreases under a condition higher than pH 7, and thus it is difficult to formulate brimonidine into an aqueous solution having a transparent property at a pH of more than 7 to 8 or less in a biocompatible range.

In particular, it may be considered to increase the content of the active ingredient in order to secure a further increased therapeutic effect and convenience of taking in the ophthalmic preparation, but in the case of brimonidine, it is difficult to sufficiently dissolve brimonidine within a biocompatible pH range, and thus there is a limit to the development of the ophthalmic preparation containing a high concentration of brimonidine while maintaining biocompatibility.

### <Related Art References>

### <Patent Documents>

Korea Unexamined Patent Application Publication No. 10-2003-0017500

### Detailed Description of the Invention

### Technical Problem

The present invention may provide an ophthalmic composition including brimonidine or salts thereof, a preventive or therapeutic method using the same, and a use thereof, in which the composition includes brimonidine stably dissolved therein, may be safely administered to eyeballs, and may maintain a stable state during storage without the occurrence of precipitation and a change in appearance thereof.

### Technical Solution

An ophthalmic composition according to the present invention may include brimonidine or salts thereof; sulfobutylether-β-cyclodextrin (SBE-β-CD) or salts thereof; and xanthan gum.

In embodiments of the present invention, "brimonidine" may refer to a compound having a chemical name of 5-bromo-6-(2-imidazolidinylideneamino)quinoxaline.

In embodiments of the present invention, the salts of brimonidine may include brimonidine tartrate.

In embodiments of the present invention, the ophthalmic composition may include brimonidine or salts thereof in an amount of about 0.01 w/v% or more, about 0.1 w/v% or more, about 0.2 w/v% or more, or about 0.3 w/v% or more, and in an amount of about 10 w/v% or less, about 5 w/v% or less, about 1 w/v% or less, about 0.9 w/v% or less, about 0.8 w/v% or less, about 0.7 w/v% or less, about 0.6 w/v% or less, or about 0.5 w/v% or less based on the total volume of the ophthalmic composition. For example, the ophthalmic composition may include brimonidine or salts thereof in an amount of about 0.01 to about 10 w/v%, about 0.1 to about 5 w/v%, about 0.1 to 1 w/v%, about 0.2 to about 1 w/v%, about 0.3 to about 1 w/v%, about 0.2 to about 0.5 w/v%, about 0.3 to about 0.5 w/v%, or about 0.1 to about 0.5 w/v%.

In one embodiment, the ophthalmic composition may include brimonidine or salts thereof in an amount of about 0.2 w/v% or more based on the total volume of the ophthalmic composition. As one example, the ophthalmic composition may include brimonidine or salts thereof in an amount of about 0.2 to about 1 w/v%, or about 0.3 to about 1 w/v%.

In one embodiment, the ophthalmic composition may include brimonidine or salts thereof in an amount of about 0.3 w/v% or more, and in an amount of about 0.5 w/v% or less based on the total volume of the ophthalmic composition. As one example, the ophthalmic composition may include brimonidine or salts thereof in an amount of about 0.3 to about 0.5 w/v%.

In embodiments of the present invention, the salts of SBE-β-CD are not particularly limited, but may include a metal salt. For example, the salt of SBE-β-CD may be a sodium salt.

In embodiments of the present invention, the ophthalmic composition may include SBE-β-CD or salts thereof in an amount of about 1 w/v% or more, about 3 w/v% or more, about 5 w/v% or more, or about 6 w/v% or more, and in an amount of about 15 w/v% or less, about 10 w/v% or less, or about 8 w/v%. For example, said ophthalmic composition may include SBE-β-CD or salts thereof in an amount of about 1 to about 15 w/v%, about 1 to about 10 w/v%, about 3 to about 10 w/v%, about 5 to about 10 w/v%, about 6 to about 10 w/v%, about 5 to about 8 w/v%, or about 6 to about 8 w/v%.

In embodiments of the present invention, the ophthalmic composition may include xanthan gum in an amount of about 0.01 w/v% or more, or about 0.1 w/v% or more, and in an amount of about 10 w/v% or less, about 6 w/v% or less, about 3 w/v% or less, about 1 w/v% or less, about 0.8 % or less, or about 0.6 w/v% or less. For example, the ophthalmic composition may include xanthan gum in an amount of about 0.01 to about 10 w/v%, about 0.01 to about 6 w/v%, about 0.1 to about 3 w/v%, about 0.1 to about 1 w/v%, about 0.1 to about 0.6 w/v%, or about 0.01 to about 1 w/v%.

According to the ophthalmic composition of the present invention, SBE-β-CD or salts thereof and xanthan gum may be included to stably and sufficiently dissolve brimonidine or salts thereof in a preparation process and to maintain a stable state without the occurrence of precipitation and a change in appearance during storage.

In embodiments of the present invention, the ophthalmic composition may have a pH of about 7.2 or more, about 7.3 or more, or about 7.4 or more, and may have the pH of about 9 or less, or about 8 or less. For example, the ophthalmic composition may have the pH of about 7.2 to about 9, about 7.2 to about 8, about 7.3 to about 8, or about 7.4 to about 8.

In embodiments of the present invention, the ophthalmic composition may include about 0.2 w/v% or more of brimonidine or salts thereof; SBE-β-CD or salts thereof; and xanthan gum.

In embodiments of the present invention, the ophthalmic composition may include about 0.3 w/v% or more of brimonidine or salts thereof; SBE-β-CD or salts thereof; and xanthan gum.

In embodiments of the present invention, the ophthalmic composition may include brimonidine or salts thereof; SBE-β-CD or salts thereof; and xanthan gum, and may have the pH of about 7.2 to about 8.

In embodiments of the present invention, the ophthalmic composition may include about 0.2 w/v% or more of brimonidine or salts thereof; SBE-β-CD or salts thereof; and xanthan gum, and may have the pH of about 7.2 to about 8.

In embodiments of the present invention, the ophthalmic composition may include about 0.3 w/v% or more of brimonidine or salts thereof; SBE-β-CD or salts thereof; and xanthan gum, and may have the pH of about 7.4 to about 8.

In embodiments of the present invention, the ophthalmic composition may include brimonidine or salts thereof in an amount of about 0.3 to about 0.5 w/v%; SBE-β-CD or salts thereof in an amount of about 1 to about 10 w/v%; and xanthan gum in an amount of about 0.1 to about 0.6 w/v%, and may have the pH of about 7.2 to about 8.

In embodiments of the present invention, the ophthalmic composition may include an additive such as a buffering agent, pH adjuster, dissolution aid, sustained release agent, thickener, isotonic agent, and the like.

In embodiments of the present invention, the buffering agents used herein may include at least one of acetic acid, phosphoric acid, boric acid, salts thereof, and/or hydrates or anhydrides thereof, etc., but is not limited thereto. The buffering agent may be added in an amount appropriate to maintain an appropriate pH.

In embodiments of the present invention, the pH adjusters used herein may include hydrochloric acid, sodium hydroxide, or the like. The pH adjuster may be used in an amount necessary to obtain an appropriate range of pH.

In embodiments of the present invention, the ophthalmic composition may include polysorbate 20, polysorbate 80, and the like as the dissolution aid.

In embodiments of the present invention, the ophthalmic composition may include polyvinylpyrrolidone, cellulose-based compounds, and the like as the thickener.

The ophthalmic composition of the present invention may be a solution having a transparent appearance including water. In embodiments of the present invention, water used as an aqueous medium may be sterile purified water, water for injection, and the like, which is suitable for preparing the ophthalmic preparation. In one embodiment, the ophthalmic composition of the present invention may be an aqueous ophthalmic composition including water.

In embodiments of the present invention, the ophthalmic composition may be a transparent and stable solution continuously for at least three months upon storage at a temperature of about 20°C to about 40°C.

In embodiments of the present invention, said ophthalmic composition may be a transparent and stable solution continuously for at least two weeks upon storage at a temperature of about 70°C.

In embodiments of the present invention, the ophthalmic composition may include brimonidine or salts thereof as an active ingredient, and may exhibit pharmacological activity of brimonidine or salts thereof. Particularly, the ophthalmic composition may be advantageously used for preventing, ameliorating or treating glaucoma and/or ocular hypertension.

In embodiments of the present invention, the ophthalmic composition may further include an additional active ingredient in addition to brimonidine or salts thereof. Examples of the additional active ingredient may include latanoprost, bimatoprost, travoprost, tafluprost, netarsudil, carteolol, timoptic, timolol, betaxolol, levobunolol, metipranolol, brinzolamide, dorzolamide, acetazolamide, methazolamide, or pharmaceutically acceptable salts thereof, each of which may be used alone or two or more of which may be used together in combination with brimonidine or salts thereof.

In embodiments of the present invention, the ophthalmic composition may be intended for single-use or multi-use, and particularly single use. When the ophthalmic composition is for multi-use, the composition may further include a preservative. The preservative may be a quaternary ammonium compound such as benzalkonium chloride, polyquaternium-1 (e.g., polyquad), and the like; a guanidine-based compound such as chlorohexidine, and the like; chlorobutanol; a mercury preservative, and the like.

In embodiments of the present invention, the ophthalmic composition may be administered once to three times a day. For example, the ophthalmic composition may be administered once, twice, or three times a day.

The ophthalmic composition of the present invention may include brimonidine, which is stably dissolved therein, and thus may be provided as an aqueous liquid having a transparent appearance. In addition, the ophthalmic composition of the present invention may maintain a stable state without occurrence of precipitation and a change in the appearance such as color or the like, during storage. Furthermore, the ophthalmic composition may have excellent stability in that the physicochemical properties thereof such as the content of the active ingredient, the pH and the like are well maintained, and a small amount of impurities is generated. Thus, the ophthalmic composition of the present invention may maintain a stable state for a long period of time, and thus may be safely administered to eyeballs and may exhibit an excellent therapeutic effect without side effects.

The above-described ophthalmic composition according to the present invention may be a composition for lowering an intraocular pressure.

The above-described ophthalmic composition according to the present invention may prevent or treat at least one of glaucoma and ocular hypertension.

The above-described ophthalmic composition according to the present invention may lower an intraocular pressure of a subject who has at least one of glaucoma and ocular hypertension.

In the present invention, the "subject" may refer to all animals including humans.

The present invention may provide a method for preventing or treating at least one of glaucoma and ocular hypertension, which includes administering the above-described ophthalmic composition to a subject.

The present invention may provide a use of the above-described ophthalmic composition for preventing or treating at least one of glaucoma and ocular hypertension.

The present invention may provide a use of the above-described ophthalmic composition for preparing a medication for preventing or treating at least one of glaucoma and ocular hypertension.

Matters mentioned in the ophthalmic composition, use, and method for treating or preventing diseases of the present invention may be equally applied, if not contradictory to each other.
(1) An ophthalmic composition according to the present invention may include brimonidine or salts thereof; SBE-β-CD or salts thereof; and xanthan gum.
(2) The ophthalmic composition according to above (1) may include brimonidine or salts thereof in an amount of about 0.2 w/v% or more based on the total volume of the composition.
(3) The ophthalmic composition according to above (1) or (2) may include brimonidine or salts thereof in an amount of about 0.2 w/v% to about 1 w/v% based on the total volume of the composition.
(4) The ophthalmic composition according to any one of above (1) to (3) may include brimonidine tartrate as a salt of brimonidine.
(5) The ophthalmic composition according to any one of above (1) to (4) may include SBE-β-CD or salts thereof in an amount of about 1 to about 15 w/v% based on the total volume of the composition.
(6) The ophthalmic composition according to any one of above (1) to (5) may include xanthan gum in an amount of about 0.01 to about 1 w/v% based on the total volume of the composition.
(7) The ophthalmic composition according to any one of above (1) to (6) may have the pH of about 7.2 to about 8.
(8) The ophthalmic composition according to any one of above (1) to (7) may be a transparent solution including water.
(9) The ophthalmic composition according to any one of above (1) to (8) may include at least one additive selected from a buffering agent, pH adjuster, dissolution aid, sustained release agent and thickener.
(10) The ophthalmic composition according to any one of above (1) to (9) may be a transparent solution continuously for at least three months upon storage at a temperature of about 20°C to about 40°C, or a transparent solution continuously for at least two weeks upon storage at a temperature of about 70°C.
(11) The present invention may provide a method for preventing or treating at least one of glaucoma and ocular hypertension, which includes administering the ophthalmic composition to a subject. In this case, the ophthalmic composition may be an ophthalmic composition according to any one of above (1) to (10).
(12) The present invention may provide a use of the ophthalmic composition for preventing or treating at least one of glaucoma and ocular hypertension. In this case, said ophthalmic composition may be an ophthalmic composition according to any one of above (1) to (10).
(13) The present invention may provide a use of the ophthalmic composition for preparing a medication for preventing or treating at least one of glaucoma and ocular hypertension. In this case, the ophthalmic composition may be an ophthalmic composition according to any one of above (1) to (10).

The method for preparing the ophthalmic composition according to the present invention may include preparing a mixed solution including brimonidine or salts thereof, SBE-β-CD or salts thereof, and xanthan gum.

In embodiments of the present invention, the preparing of the mixed solution may be performed by dissolving brimonidine or salts thereof, SBE-β-CD or salts thereof, and xanthan gum in water.

In embodiments of the present invention, said mixed solution may be a solution subjected to a sterilization process.

In embodiments of the present invention, the preparing of the mixed solution may include:
preparing a first solution by dissolving brimonidine or salts thereof and SBE-β-CD or salts thereof in water;
preparing a second solution by dissolving xanthan gum in water; and
mixing the first solution and the second solution.

In one embodiment, the first solution and the second solution may be subjected to a sterilization process, and then mixed to prepare said mixed solution.

In embodiments of the present invention, the first solution, the second solution, and the mixed solution in preparing the mixed solution may each independently further include an additive such as a buffering agent, pH adjuster, dissolution aid, sustained release agent, thickener, isotonic agent, and the like.

In embodiments of the present invention, at least one of the first solution, the second solution and the mixed solution in preparing the mixed solution may further include an additional active ingredient in addition to brimonidine or salts thereof.

The method for preparing the ophthalmic composition according to the present invention may include adding the pH adjuster to the mixed solution.

In the method for preparing the ophthalmic composition according to the present invention, brimonidine or salts thereof, SBE-β-CD or salts thereof, and xanthan gum, as well as additional additives and additional active ingredients may be substantially the same as described above, and thus any redundant detailed description will be omitted.

### Advantageous Effects

The ophthalmic composition of the present invention may include brimonidine, which is stably dissolved therein, and thus may be provided as an aqueous solution having a transparent appearance.

In addition, the ophthalmic composition of the present invention may maintain a stable state without occurrence of precipitation and a change in the appearance such as color or the like, during storage.

Furthermore, the ophthalmic composition may have excellent stability in that the physicochemical properties thereof such as the content of the active ingredient, the pH and the like are well maintained, and a small amount of impurities are generated.

Thus, the ophthalmic composition of the present invention may be safely administered to eyeballs and maintain a stable state for a long period of time, and thus may exhibit an excellent therapeutic effect without side effects.

### Brief Description of the Drawings

FIG. 1 is a view showing the results of Experimental Example 1.
FIG. 2 is a view showing the results of Experimental Example 2.

### Mode for Invention

Hereinafter, the present invention will be described in more detail through experimental examples. These experimental examples are provided only for the purpose of illustrating the present invention, and thus it will be apparent to those skilled in the art that the scope of the present invention is not limited thereto.

### Preparation Example 1: Preparation of ophthalmic compositions 1 to 3

Compositions 1 to 3 were prepared in accordance with the ingredients and contents as shown in table 1 below.

Particularly, brimonidine tartrate, sodium phosphate dibasic anhydrous, sodium dihydrogen phosphate monohydrate, and SBE-β-CD sodium were dissolved in water, and then subjected to a filter sterilization process to prepare a first solution. Separately, xanthan gum was dissolved in water and subjected to a high-temperature and high-pressure sterilization process to prepare a second solution. Composition 1 was prepared by mixing the first solution and the second solution and adjusting the pH with sterile sodium hydroxide.

Composition 2 was prepared in the same manner as in Composition 1, except for SBE-β-CD sodium.

Composition 3 was prepared by preparing the first solution subjected to the aseptic process and adjusting the pH with sodium hydroxide.

The prepared compositions 1 to 3 had the pH of about 7.2.

**[Table 1]**

| Ingredient | Content (w/v%) | | |
|---|---|---|---|
| | Composition 1 | Composition 2 | Composition 3 |
| Brimonidine tartrate | 0.4 | 0.4 | 0.4 |
| SBE-β-CD sodium | 6 | - | 6 |
| Xanthan gum | 0.2 | 0.2 | - |
| Sodium phosphate dibasic anhydrous | 1.0 | 1.0 | 1.0 |
| Sodium dihydrogen phosphate monohydrate | 0.027 | 0.027 | 0.027 |
| Sodium hydroxide | q.s. | q.s. | q.s. |
| Water (water for injection) | q.s. | q.s. | q.s. |

### Experimental Example 1: Appearance & stability evaluation

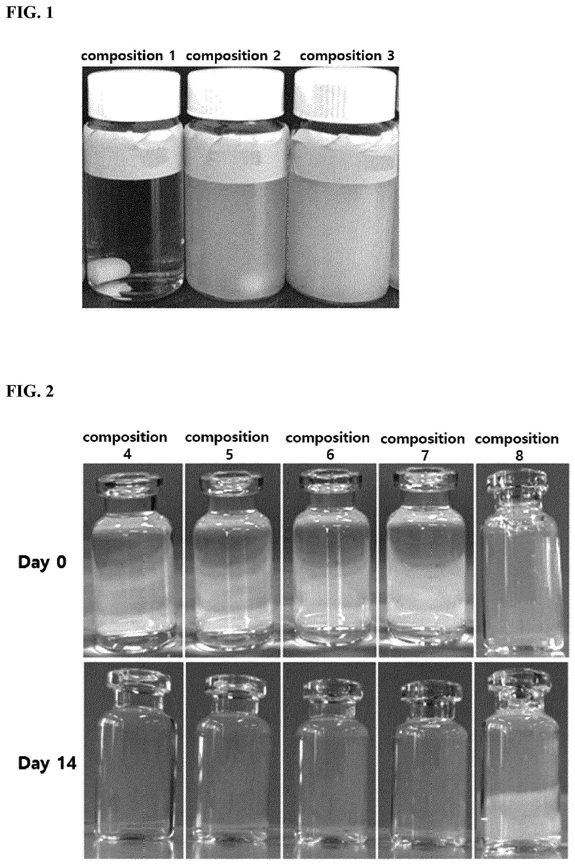

It was confirmed that compositions 2 and 3 cause precipitation immediately after preparation thereof. In contrast, composition 1 was obtained as a transparent solution (FIG. 1).

In addition, composition 1 was stored at room temperature (25°C), 30°C and 40°C for three months, respectively, and at 70°C for two weeks, and was observed for precipitation. As a result, it was confirmed that a transparent appearance is maintained without precipitation under all of the above storage conditions.

Through this, it can be seen that composition 1 including SBE-β-CD and xanthan gum of the present invention has very excellent stability.

### Preparation Example 2: Preparation of ophthalmic compositions 4 to 8

Compositions 4 to 8 were prepared in accordance with the ingredients and contents as shown in table 2 below by means of the same method as the method for preparing composition 1.

**[Table 2]**

| Ingredient | Content (w/v%) | | | | |
|---|---|---|---|---|---|
| | Composition 4 | Composition 5 | Composition 6 | Composition 7 | Composition 8 |
| Brimonidine tartrate | 0.1 | 0.2 | 0.3 | 0.5 | 0.5 |
| SBE-β-CD sodium | 1 | 3 | 5 | 8 | 10 |
| Xanthan gum | 0.1 | 0.2 | 0.3 | 0.6 | 0.6 |
| Sodium phosphate dibasic anhydrous | - | - | - | - | 0.5 |
| Sodium dihydrogen phosphate anhydrous | 0.5 | 0.5 | 0.5 | 0.5 | 0.5 |
| Sodium hydroxide | q.s. | q.s. | q.s. | q.s. | q.s. |
| Water for injection | q.s. | q.s. | q.s. | q.s. | q.s. |
| pH | 8 | 7.8 | 7.59 | 7.4 | 7.83 |

### Experimental Example 2: Appearance & stability evaluation

Compositions 4 to 8 were all prepared as transparent solutions, and there was no composition in which precipitation occurred.

In addition, it was confirmed that all of compositions 4 to 8 maintain a transparent appearance without color change and occurrence of precipitation even when stored at 70°C for two weeks (FIG. 2).

Through this, it can be seen that the composition including SBE-β-CD and xanthan gum of the present invention has very excellent stability.

### Experimental Example 3: Stability evaluation

The composition 6 was stored at 70°C for one week, and then the pH, osmotic pressure, and viscosity were measured. The viscosity was measured using a rotational viscometer (Brookfield viscometer) at a rate at which a torque value was as much as 100% as possible under the conditions of spindle 18 and 25±0.5°C.

It was confirmed that the pH, osmotic pressure, and viscosity of composition 6 remain substantially stable without change even after storage (Table 3).

**[Table 3]**

| | **pH** | **Osmolality (mOsmol/kg)** | **Viscosity (cP·s)** |
|---|---|---|---|
| Day 0 | 7.59 | 243 | 50.8 |
| Day 3 | 7.39 | 245 | 49.9 |
| Day 7 | 7.32 | 248 | 50.4 |

Through this, it can be seen that the composition including SBE-β-CD and xanthan gum of the present invention has very excellent stability.

While specific portions of the present invention have been described in detail above, it is apparent to those skilled in the art that such detailed descriptions are set forth to illustrate exemplary embodiments only, but are not construed to limit the scope of the present invention. Thus, it should be understood that the substantial scope of the present invention is defined by the accompanying claims and equivalents thereto.

## Claims

1. An ophthalmic composition comprising:
brimonidine or salts thereof;
sulfobutylether-β-cyclodextrin (SBE-β-CD) or salts thereof; and
xanthan gum.

2. The ophthalmic composition of claim 1, wherein brimonidine or salts thereof are comprised in an amount of 0.2 w/v% or more based on a total volume of the composition.

3. The ophthalmic composition of claim 2, wherein brimonidine or salts thereof are comprised in an amount of 0.2 to 1 w/v% based on a total volume of the composition.

4. The ophthalmic composition of claim 1, wherein brimonidine tartrate is comprised as a salt of brimonidine.

5. The ophthalmic composition of claim 1, wherein SBE-β-CD or salts thereof are comprised in an amount of 1 to 15 w/v% based on a total volume of the composition.

6. The ophthalmic composition of claim 1 or 5, wherein xanthan gum is comprised in an amount of 0.01 to 1 w/v% based on a total volume of the composition.

7. The ophthalmic composition of claim 1 or 2, wherein pH is 7.2 to 8.

8. The ophthalmic composition of claim 1, wherein the ophthalmic composition is a transparent solution comprising water.

9. The ophthalmic composition of claim 1, wherein the ophthalmic composition comprises at least one additive selected from a buffering agent, pH adjuster, dissolution aid, sustained release agent and thickener.

10. The ophthalmic composition of claim 1, wherein the ophthalmic composition is a transparent solution continuously for at least three months upon storage at a temperature of 20°C to 40°C, or a transparent solution continuously for at least two weeks upon storage at a temperature of 70°C.

11. A method for preventing or treating at least one of glaucoma and ocular hypertension, the method comprising
administering the ophthalmic composition according to any one of claims 1 to 10 to a subject.

12. A use of the ophthalmic composition according to any one of claims 1 to 10 for preventing or treating at least one of glaucoma and ocular hypertension.

13. A use of the ophthalmic composition according to any one of claims 1 to 10 for preparing a medication for preventing or treating at least one of glaucoma and ocular hypertension.
